# EUROPEAN PATENT APPLICATION

(11) **EP 0 775 467 A1**
(43) Date of publication of application: **28.05.1997**
(21) Application number: 96660087.6
(22) Date of filing: 18.11.1996
(51) Int. Cl.: A61B 6/00

(54) **Method and system for controlling the functions of a mammography apparatus**

(30) Priority: 23.11.1995 FI 955636
(71) Applicant: PLANMED OY, 00810 Helsinki (FI)
(72) Inventor: Strömmer, Pekka, 02210 Espoo (FI)
(74) Representative: Pekonen, Raimo Kalevi

(57) **Abstract**

The invention relates to a method and system for controlling the functions of a mammography apparatus, in which control method the mammography apparatus is used to make radiographic exposures in different projections from breasts (M) to be examined. The method uses a control system (10) having a memory (16) into which, prior to the exposure, projection parameter information is stored. On the basis of this information, a control unit (15) of the control system (10) steers actuator motors (31,33,34,35, 40) of the mammography apparatus so as to carry out the movements of the different actuators (38,43,44,45,47,48,100) driven by the actuator motors automatically in a continuous sequence and/or subsequences activated by the operator of the mammography apparatus. The invention is particularly suited for use in screening examinations of breast cancer.

## Description

The present invention relates to a method of controlling the functions of a mammography apparatus, whereby the mammography apparatus is controlled according to the method to make exposures from the breasts to be radiographed using different projections.

The invention further relates to a control system of a mammography apparatus comprising a base, a movable, preferably about the horizontal axis rotatable C-arm or similar member connected thereto, said C-arm carrying an x-ray source, compression paddles for the breast to be radiographed, and imaging means, and said mammography apparatus further comprising actuator motors of the movable members of the apparatus.

Mammography as an x-ray radiographic technique is the most important diagnostic method for finding breast cancer in women. The earlier a cancer can be identified, the better the odds of its cure. Therefore, mammography equipment is today subject to stringent demands on high image resolution to facilitate the recognition of a possible cancer already in its initial stage on the basis of, e.g., microcalcifications.

A commonly used approach to finding breast cancers is to perform mammographic screening campaigns in which women of a given age group are called at constant intervals to mammography in order to identify malignant breast tumors. The large number of women to be radiographed also requires the use of high-quality mammography equipment particularly in terms of throughput speed.

In mammographic screening, two exposures are conventionally taken from each breast, that is, a total of four exposures per patient. Sometimes also a few additional exposures are taken, either as a precaution or to rectify some failure in a previous session. In general, the throughput of mammographic screening stations is assumed as about ten patients per hour, which means taking about 40 exposures per hour, whereby the time available for a single exposure is only of the order of one and half minutes. During this time, the radiography apparatus must be aligned into a correct position depending on the shape of the breast and the projection to be viewed therefrom, the patient must further be supported in the apparatus and the breast to be radiographed compressed, then the exposure taken, the breast to be uncompressed, the film cassette changed - unless a digital camera is used - and the radiography apparatus realigned for the next exposure.

On the basis of the foregoing it is obvious that operation at a mammography station is strenuous and hasty, whereby any measure that can moderate the stress and workload of the radiography apparatus operator and increase the throughput speed of screening examinations is welcome.

Conventionally, an aid to the above-described problems has been sought from improvements in work methods and addition of properties to mammography equipment that can speed up operations. Such known properties are, e.g., motor-actuated movements which reduce the operator's physical workload by performing the positioning of the heavy moving components of the mammography apparatus without requiring external actuation by manual force. For mammography, the art knows a plurality of different compression methods developed to aid and speed the positioning of the breast, and the constructions of modern equipment have been optimized to assist operation, whereby in isocentric rotation, for instance, the patient need not be transferred laterally nor any significant apparatus height adjustments are required when changing from one projection to another. With regard to the state of the art concerning a mammography apparatus implementing said isocentric rotation and other matter related to the present invention, reference is made to FI Pat. No. 80,996 (corresponding US Pat. No. 5,050,197), filed by the applicant.

Under years of practice, operators performing mammographic screening examinations on a continuous basis have gathered their own experiences and concepts how to carry out exposures in the fastest and least strenuous manner. One of these factors of personal preference is the order in which the radiographic images from the two breasts are taken and which projections are to be used. While the order of exposures varies slightly according to the differing experiences and habits of the radiography apparatus operators, each operator typically adheres to a certain sequence offering fastest and easiest operation during the mammography session.

Modern mammography apparatus are extensively motorized to a degree relieving the operator from using a great amount of manual force to adjust the apparatus to the positions required by the different projections. Yet, these operations still take a considerable time, because after each exposure the operator must change the film cassette, unless an up-to-date rare digital imaging system is available, and the C-arm and height of the unit must be adjusted for the next exposure, possibly also the exposure parameters of the apparatus needing fine-tuning.

The present invention aims to offer novel approaches to the above-discussed problems so that such prior-art drawbacks and others to be mentioned later will be essentially overcome.

It is a broad object of the present invention to provide such a method and system for controlling the functions of a mammography apparatus that can facilitate mammography and particularly mammographic screening performed using a plurality of different projections so as to make the exposures easier and less stressing for both the apparatus operator and the patient.

It is a nonlimiting further object of the invention to facilitate when necessary a higher making rate of exposures, particularly in mammographic screening, without compromising the quality or diagnostic value of the radiographs.

To achieve the above-stated goals and others to be explained later, the method according to the invention is principally characterized in that the method employs a control system into the memory of which, prior to the start of the exposure, are stored at least the projection parameters of the exposure, on the basis of which the control unit of the control system drives the actuator motors of the mammography apparatus so as to carry out the movements of the different actuators in a continuous sequence, or alternatively, in subsequences activated under the control of the mammography apparatus operator.

Furthermore, the apparatus according to the invention is principally characterized in that said control system comprises a memory and a control unit, whereby the control signals of said control unit can be used for steering the function of said actuator motors so that, on the basis of projection parameters stored in said memory, the movements of the different actuators in the mammography apparatus can be carried out automatically and/or in subsequences activated under the control of the mammography apparatus operator.

By virtue of the control method and unit according to the invention, the projection parameters of the next series of exposures in the sequence can be preset in the memory of the control system. The projection parameters can be entered in the mammography equipment itself as projections or position parameters, or alternatively, they can be entered in a separate computer or similar apparatus, which during the intervals between exposures transfers the position information on the next exposure to the mammography equipment either by directly driving the apparatus into the preset position or by providing the radiography apparatus with the projection information, on the basis of which the apparatus itself subsequently controls its actuators to attain the correct position.

The function of the method and control unit according to the invention is such that each operator working in a certain radiographic station first presets the sequence of projections only once into the control system, and when starting the sequence of exposures, launches this sequence of personal preference as simply as by depressing a single key. Here, the apparatus making the exposures from the patient performs self-sustained actuation of its correct positioning after each exposure to the projection parameters of the next exposure, either by automatically driving the actuators into the correct position of the next exposure immediately after the patient's breast is released from the compression of the apparatus, or alternatively, so that after the completion of the previous exposure, the operator assures correct release of the patient from the apparatus and verifies this condition by depressing a key, whereby the apparatus is enabled to drive its actuators to the correct position for the next exposure. As an additional safety measure, the apparatus is provided with an indication of which breast is to be compressed for next exposure.

During the automatic mode of actuator positioning, as the apparatus is aligning itself automatically according to the preset parameters for the next exposure, the operator can utilize the meantime for, e.g., changing the film cassette or preparing the patient for the exposure.

The programming and other facilities applied in the invention may be located in the mammography apparatus or its control computer. When a plurality of operators will be using the same mammography apparatus, each operator may have different operator-specified sequences stored for varying radiography situations.

Additionally, the system according to the invention advantageously includes facilities for easy entry of parameters for single exposures, that is, when necessary permitting a deviation from the preset sequence even amidst an uncompleted sequence. A further advantageous property of the system is a facility of bidirectional random selection of exposures in the exposure sequence so that, e.g., after an error has occurred in the previous exposure, a re-exposure of the view may be made even if a few exposures were made inbetween, since the detection of an error may take several minutes due to the processing delay of the exposed film.

In the following the invention will be described in greater detail by making reference to the diagrams of appended drawings illustrating diagrammatically a few exemplifying embodiments of the invention, whereby the details of the diagrams must not be understood limiting the scope of the invention, in which drawings
Figure 1 is a perspective view of an exposure-ready mammography apparatus capable of utilizing the control method and unit according to the invention;
Figures 2-6 are a series of diagrams illustrating viewing projections capable of being controlled by the method and control unit according to the invention;
Figure 7 is a schematic block diagram of an embodiment implementing the control method and system according to the invention in an application based on digital imaging and a CCD-sensor-based imaging system; and
Figure 8 is a schematic block diagram equivalent to that shown in Fig. 7, now adapted for an operating environment based on using an x-ray film as the recording medium.

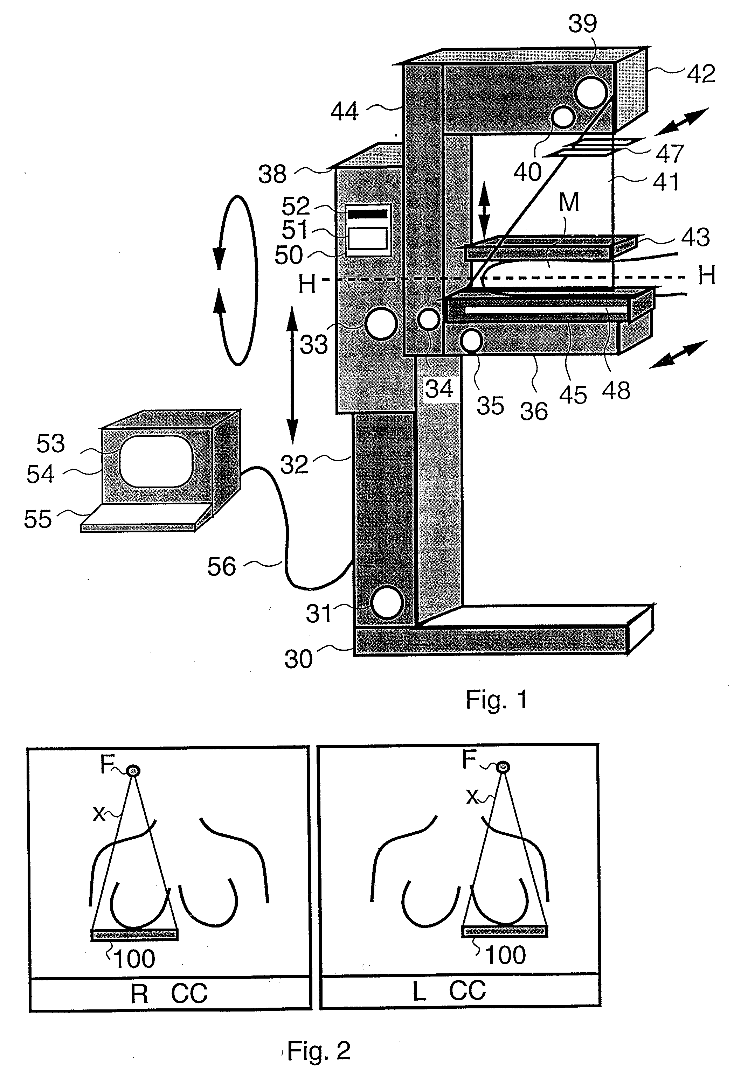

Referring to Fig. 1, a mammography apparatus suitable for use as an application platform for the present invention is shown therein standing on a base 30. To the base 30 is attached a fixed vertical column section 32 housing a lift motor 31 by means of which a movable vertical column section 38 can be telescopically elevated/lowered inside the fixed column section. The vertical column section 38 houses a motor 33 for rotating a C-arm 44 about a horizontal axis H-H. One end of the C-arm supports an x-ray generator 42, while the other end of the arm carries a shelf-like lower breast support 36 against which a breast M to be radiographed is compressed with the help of a compressing paddle-like upper support 43 actuated by a motor 34. The compressing support 43 is advantageously designed into a pivoted and tilting paddle support by means of which the breast being radiographed is compressed at the upper part of its basal perimeter with the help of the tip of the paddle 43 and the paddle 43 is then rotated about its pivotal point until aligned essentially parallel with the lower support 36. With regard to this construction and other details related to the implementation of the mammography apparatus, reference is made to FI Pat. No. 80,996 (corresponding US Pat. No. 5,050,197), filed by the applicant. During the exposure of the breast M, a narrow x-ray beam 41 emitted by an x-ray tube 39 passes through a primary blind 47, which is adapted laterally movable by means of a motor 40, then passes through the breast M being radiographed and finally is incident on a digital sensor head 45, which is adapted simultaneously movable with the primary blind by means of a motor 35 and may be implemented in the same fashion as the CCD sensor system disclosed in FI Pat. Appl. 955,598 (filed on Nov. 21, 1995) and being freely configurable within given limits under program control. Obviously, the invention may also be adapted for use in a mammography apparatus having the digital imaging sensor head 45 replaced by a processable film, whereby the entire imaged area is radiographed by a single exposure.

Reference numeral 48 denotes either a film cassette or a digital imaging sensor head, respectively, and reference numeral 47 denotes either a slit blind driven by a motor 40, or a conventional beam-limiting collimator having its window size controlled by means of an actuator motor 40, respectively.

The mammography apparatus shown in Fig. 1 has a control panel 50 incorporating a keypad 51 and a display 52. There may be a number of the control panels 50 connected to the same mammography unit, most advantageously adapted to both sides of the vertical column section 38 of the apparatus base part. The apparatus may also be controlled by a computer 54 or equivalent control unit having a keypad 55 and a display 53. The computer 54 is connected to the mammography apparatus via a cable 56. The control of the apparatus may be arranged to occur from either of the keypads 51,55 or from both.

Referring to Figs. 2-6, the most common imaging projections of the breasts that are also frequently used in radiographic screening examinations. In Fig. 2 is shown projection CC (Cranio-Caudal) of the right breast R and the left breast L, wherein the radiation beam X is emitted by the tube focus F, passes through the breast and impinges on the imaging means 100. This projection is essentially vertical. While extremely rarely used, a modification of this projection known as the Cleopatra view may be used having the plane of the projection inclined by 5°.

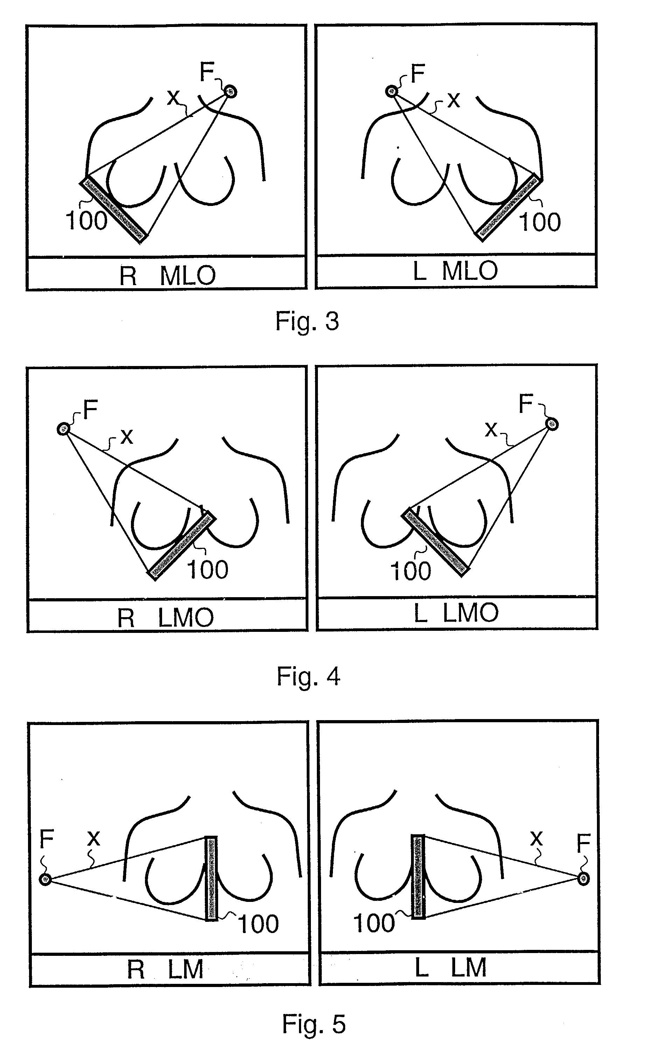

In Fig. 3 are shown the MLO (Medio-Lateral Oblique) projections, which depending on the convention varying in different countries are taken at an angle of 45°-65° (whereby 0° angle corresponds to the vertical plane), however, using essentially the same angle for the exposures from both breasts. The term "Medio Lateral" refers herein to the x-ray beam alignment, wherein the rays pass through the tissues from the center of the patient toward the side.

In Fig. 4 is shown the same projection as in Fig. 3, now called the LMO, or Latero-Medial Oblique projection, since herein the propagation direction of the x-ray beam is from the side of the patient toward the center.

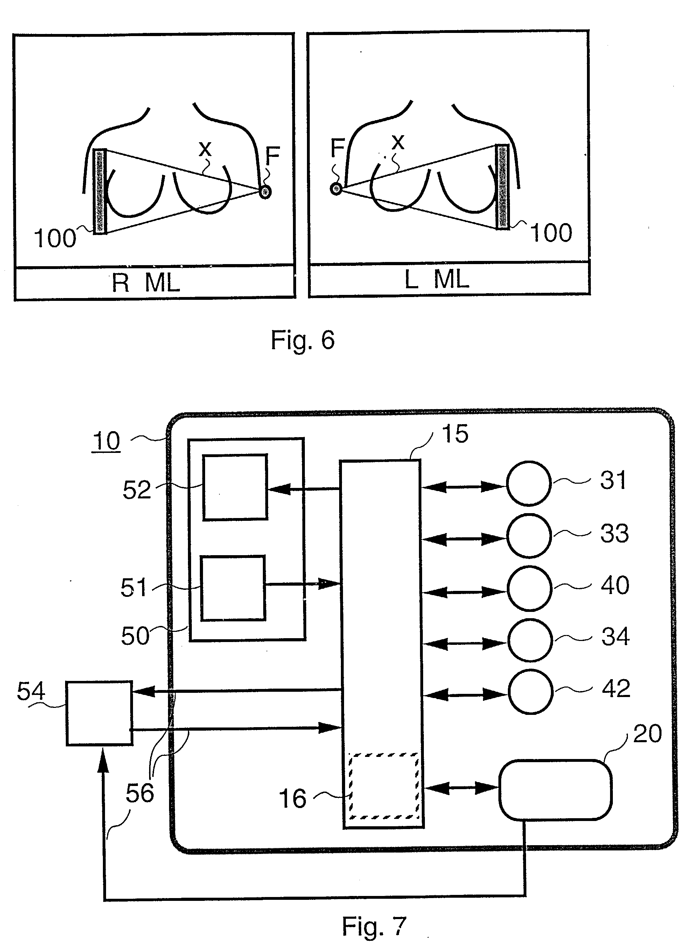

In Fig. 5 is shown the LM, or Latero-Medial projection, which is taken at 90° angle. In Fig. 6, respectively, is shown the Medio-Lateral projection.

Conventionally, two different lines of practice are used in screening examinations depending on the country. If the exposures are made only one per each breast, the projection used is MLO (see Fig. 3), because in this projection are optimally imaged the upper outer quadrant of the breast and the armpit and the adjacent areas thereof, where breast cancer is found rather frequently. If two exposures are taken per each breast, as is conventional in Finland, the additional exposures are taken in the CC projection (see Fig. 2).

Depending on the clinical findings, additional confirming exposures may be necessary such as horizontal projections or magnifications, which are generally taken individually on a case-by-case basis. In some special occasions also an Oblique projection with the beam directed from below obliquely upward or a PA projection with the beam directed vertically from below upward may be taken. The projections of supplementary exposures are dependent on the location of the suspect focus in the breast.

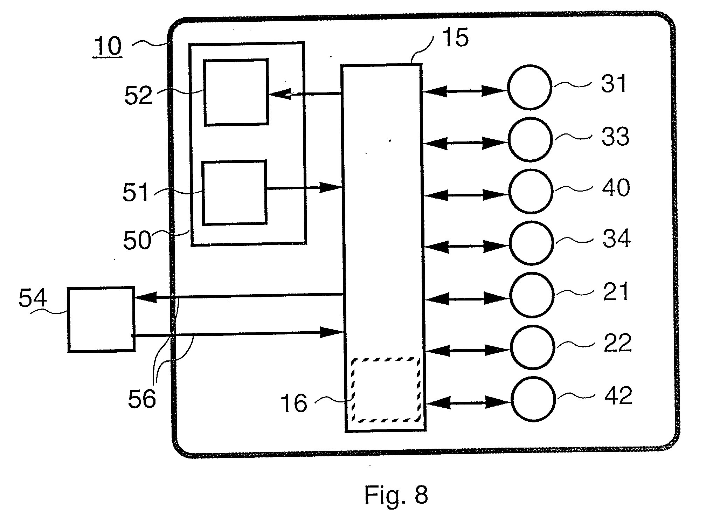

In Figs. 7 and 8 are shown block diagrams of control system implementations according to the invention suitable for use in conjunction with the mammography apparatus illustrated in Fig. 1.

The control system 10 includes a control panel 50 shown in Fig. 1 with the keypad 51 and the display 52, together with the external computer 54 connected by the cable 56 to the control system 10. The control system 10 is comprised of a control unit 15, which may be, e.g., a microprocessor-based electronic control unit implemented in a conventional manner having an integral or externally connected memory unit 16, into which are stored the variable and invariable control commands and other exposure parameters and information, which are entered therein according to the control method of the invention. The control unit 15 steers the different actuator motors of the mammography apparatus such as the lift motor 31 of the base unit vertical column section 38, the rotating motor 33 of the C-arm 44, the actuator motor 40 of the slit blind 47 or a window collimator and the actuator motor 34 of the breast supports 43.

In Fig. 7 is shown such an embodiment of the invention in which the imaging sensor unit 48;100 is a CCD sensor system 20, also controlled by the control unit 15. The sensor system 20 is further connected to the computer 54, and when necessary, to the display panel 50. For the advantageous implementation details of the CCD sensor system 20, reference is made to FI Patent Applications Nos. 955,597 and 955,599 (filed on Nov. 21, 1995).

In Fig. 8 is shown a block diagram equivalent to that of Fig. 7 of such an embodiment of the invention in which a conventional processable x-ray film is used for recording the exposure. The apparatus of Fig. 8 based on film recording includes a magnification drive motor 21 and a film labeling device 22, by means of which the patient and exposure parameter data are recorded in a conventional manner on the film. In the apparatus of Fig. 7 based on digital imaging, the magnification drive motor 21 and film labeling device 22 are redundant, because the patient and exposure parameter data are entered into the system via the keypad 51/55 and are shown on the display 52/53 along with the image exposed from the breast M, whereby there is no need for a separate labeling of the image. Obviously, a digital imaging system performs the storage of the patient and exposure parameter data on, e.g., a diskette along with the digitized radiograph file.

As desired, the same control system 10,54 may also be used for entering all other patient data such as length, weight, name, age, ID code and other personal data, together with the operator's name and the exposure parameters including the kV, mA, mAs, dose, projection, exposure time and other control data for the x-ray tube 39. Obviously, the latter set of parameters may be obtained automatically from the memory 16 of the control system 10.

Although for reasons of simplicity, the memory 16 of the control system 10 is in Figs. 7 and 8 shown located in the control unit 15, it is obvious that said memory 16 or a similar storage means may as well be located in conjunction with the display panel 50 and/or in the external computer 54.

The control system 10 according to the invention is advantageously designed and implemented so that the control system 10 is provided with facilities for alternative use of a conventional film cassette or a digital CCD imaging sensor system 20. The mammography apparatus may initially be procured with a window collimator and film cassette outfit, which accessories may later by replaced by a combination of a CCD imaging sensor system 20 and a slit blind, or alternatively, a reversed acquisition order may be chosen. Hence, the control system 10 is advantageously designed to anticipate the above-described change of accessories by providing the system with a retrofit facility and maximizing the flexibility of the control unit and system with regard to its easy programmability.

The program steering the system is stored in the memory 16 of the control unit 10. This program is most preferably interactive so that on the display 52 and/or 53 are shown the necessary control information and/or menus of the system as well as system status information and/or menus that report the functional state and possible malfunctions of the system, as well as propose user-selectable alternatives for rectification of system errors and/or advancement in the exposure sequence.

The control system may be programmed to contain a sufficient amount of intelligence so that the exposure sequences entered in the system will be carried out optimally also with regard to the dose received by the patient. Thus, the control system becomes a kind of expert system having a self-learning character in the sense that the all the different operators may store therein knowledge gained from the exposures.

The system is programmed most appropriately so that the preset exposure sequence may be stopped at any time and a facility of bidirectional random selection of exposures is provided so that any projection or exposure step may be skipped/ reselected. For instance, if an error has occurred in a certain exposure, a re-exposure of the view may be made even if a few exposures were made inbetween. This facility is necessitated by the fact that the processing delay of an exposed film takes several minutes.

When necessary, the control system may include different kinds of sensors and/or interlock means for sensing/reporting the functional status of the mammography apparatus and the positions of its moving element, as well as other sensor equipment capable of delivering control signals capable of steering the execution of the exposure sequence, and when necessary, providing sensor/interlock feedback information suitable for displaying on the display 52 and/or 53.

Not being limited by the above-described exemplifying embodiments, the details of the invention may be varied and deviated within the scope and inventive spirit of the annexed claims.

## Claims

1. A method of controlling the functions of a mammography apparatus, under the control of which method the mammography apparatus is used to make radiographic exposures in different projections from breasts (M) to be examined, **characterized** in that the method uses a control system (10) having a memory (16) into which, prior to the exposure, projection parameter information is stored that later is used by means of a control unit (15) of the control system (10) to steer actuator motors (31,33,34,35,40) of the mammography apparatus so as to carry out the movements of the different actuators (38,43,44,45,47,48,100) driven by the actuator motors automatically in a continuous sequence and/or subsequences activated by the operator of the mammography apparatus.

2. A method as defined in claim 1, **characterized** in that in the method a set of exposure sequences comprising a plurality of imaging projections are stored in the memory (16) of the control system (10) and that, on the basis of such a stored information, the control system (10) steers the actuator motors (31,33,34,35,40) of the mammography apparatus so as to make the mammography apparatus automatically perform exposures in the different projections (cf. Figs. 2-6).

3. A method as defined in claim 1 or 2, **characterized** in that the control system (10) is provided with a facility permitting a retrofit replacement of a radiographic recording film cassette with appropriate accessories by a digital imaging CCD sensor system (20) or similar outfit or a similar but reversed-order retrofit replacement of the radiographic imaging system.

4. A method as defined in any of claims 1 - 3, **characterized** in that in the memory (16) of the control system (10) of the mammography apparatus are stored a plurality of alternative projection sequences, most appropriately comprising the exposure sequences of the operator's personal preference.

5. A method as defined in any of claims 1 - 4, in which method a processable film is used as a radiographic recording medium, **characterized** in that the control system (10) of the mammography apparatus is used to steer the functions of a magnification control motor (21) and/or a film labeling device (22) (cf. Fig. 8).

6. A method as defined in any of claims 1 - 4, **characterized** in that in the control unit (15) of the mammography apparatus is used to control a digital imaging CCD sensor system (20) capable of delivering radiographic image information that can be transferred to a data recording, display and/or image processing apparatus (50-55) (cf. Fig. 7).

7. A method as defined in any of claims 1 - 6, **characterized** in that the control system (10) is operated using such an interactive program that can accept stopping of the preset exposure sequence and permit forward and/or backward stepping in the sequence, thereby overstepping at least one exposure step for the purpose of, e.g., making re-exposures.

8. A method as defined in any of claims 1 - 7, **characterized** in that the control system (10) is run and operated using such an interactive program that can display system status information on the display (52,53) of the apparatus, inquire desired control data and display menus and/or propose actions for continuation of the exposure sequence.

9. A method as defined in any of claims 1 - 8, **characterized** in that the control system (10) with the program run in the system is adapted into an expert system of certain intelligence level.

10. A control system for a mammography apparatus, said mammography apparatus comprising a base part (30,32,38) having thereto attached a movable, most advantageously about the horizontal axis (H-H) rotatable C-arm (44) or similar member carrying an x-ray beam source (42), supports (43,45) for compressing the breast being radiographed and radiographic image recording means (48,100) and said mammography apparatus further including actuator motors (31,33,34,35,40) of its moving parts, **characterized** in that the control system (10) comprises a memory (16) and a control unit (15) capable of providing control signals for steering the function of the actuator motors (31,33,34,35,40) so as to carry out the movements of the different actuators of the mammography apparatus automatically on the basis of projection parameter information stored in said memory (16) in a continuous sequence and/or subsequences activated by the operator of the mammography apparatus.

11. A system as defined in claim 10, **characterized** in that the system includes a control panel (50) incorporating a keyboard (51) and a display (52), said keyboard (51) being adapted for the entry of control commands for the actuator motors of the mammography apparatus and other similar parameters such as patient data into the control system (10).

12. A system as defined in claim 10 or 11, **characterized** in that the system includes an external computer (54) or similar means including a keyboard (55) and a display (53), said computer being connected by a cable (56) to the control system (10).

13. A system as defined in any of claims 10-12, said system using a digital imaging system, most appropriately a CCD sensor system (20), **characterized** in that said imaging sensor system (20) is adapted controllable by means of said control system (10) and said imaging sensor system (20) further being connected to the integral control panel (50) of the mammography apparatus and/or the external computer (54) (cf. Fig. 7).

14. A system as defined in any of claims 10-12, said system using processable film as the radiographic recording medium enclosed in a cassette adapted to be insertable under the lower breast support (45) of the mammography apparatus, or in conjunction thereof, **characterized** in that said system includes a magnification control motor (21) and/or film labelling device (22), said motor and/or means being adapted controllable by said control system (10) (cf. Fig. 8).

15. A system as defined in any of claims 10-14, **characterized** in that said control system (10) is connected to a mammography apparatus including a C-arm (44), which is adapted rotatable about the horizontal axis (H-H) and connected to a column section (38) that is adapted vertically movable along a vertical base section (32), and that the upper leg of said C-arm is adapted to carry an x-ray beam source (42), said mammography apparatus further including supports (43,45) for compressing the breast (M) being radiographed, to the lower one of which support (45) or in conjunction thereof is adapted a radiographic imaging means (48;100) such as an imaging CCD sensor system (20) or a film cassette.

16. Use of a control method as defined in any of claims 1-9 and/or a control system as defined in any of claims 10-14 for a mammography apparatus employed in breast cancer screening examinations carried out with a limited exposure session time per patient.
